# EUROPEAN PATENT SPECIFICATION

(11) **EP 1 589 995 B1**
(45) Date of publication and mention of the grant of the patent: **18.04.2007**
(21) Application number: 04706915.8
(22) Date of filing: 30.01.2004
(51) Int. Cl.: A61K 39/395, C07K 16/02, A61P 1/12, A61P 31/00

(54) **USE OF AVIAN ANTIBODIES**
VERWENDUNG VON VOGELANTIKÖRPERN
UTILISATION D'ANTICORPS AVIAIRES

(30) Priority: 30.01.2003 SE 0300213
(43) Date of publication of application: 02.11.2005
(73) Proprietor: Immun System I.M.S. AB, 751 83 Uppsala (SE)
(72) Inventor: LARSSON, Anders, S-753 09 Uppsala (SE); KOLLBERG, Hans, S-753 11 Uppsala (SE)
(74) Representative: Lindgren, Anders
(86) International application number: PCT/SE2004/000133
(87) International publication number: WO 2004/067035

(56) References cited:
- YOSHINORI MINE ET AL.: 'Chicken egg yolk antibodies as therapeutics in enteric infectious disease: A review' JOURNAL OF MEDICINE FOOD vol. 5, no. 3, 2002, pages 159 - 167, XP009008346
- DATABASE WPI Week 199348, Derwent Publications Ltd., London, GB; Class B04, AN 1993-386927, XP002992516 & ZA 9 209 143 A (FOLAN M. A.) 29 September 1993
- DATABASE BIOSIS PREV200000498871 KAMINSKI JR. M. V. ET AL.: 'Efficacy of oral anti-candida egg yolk antibody. A food not a drug.', XP002992517 & JOURNAL OF THE AMERICAN COLLEGE OF NUTRITION vol. 19, no. 5, October 2000, page 688
- CARLANDER DAVID ET AL.: 'Peroral immunotherapy with yolk antibodies for the prevention and treatment of enteric infections' IMMUNOLOGIC RESEARCH vol. 21, no. 1, 2000, pages 1 - 6, XP001038144
- MARQUARDT RONALD R. ET AL.: 'Passive protective effect of egg-yolk antibodies against enterotoxigenic Escherichia coli K88+ infection in neonatal and early-weaned piglets' FEMS IMMUNOLOGY AND MEDICAL MICROBIOLOGY vol. 23, 1999, pages 283 - 288, XP002244493

## Description

### Field of invention

This invention relates to a method for prophylaxis or therapy of enteric infections in newborn infants, prematurely born infants, infants having an immature immune system, patients suffering from temporary immunodeficiency and immunodeficiency diseases such as AIDS. More specifically to a method for prophylaxis or therapy of infections caused by Enterobacter cloaceae.

### Background of the invention

All kinds of infections are severe and sometimes life threatening in newborn infants - especially in those who are born prematurely. An effective immune system is of outmost importance to fight infections. The production of immunoglobuline is very immature in newborn infants. Thus, they have to rely solely on immunoglobulines transported to them from their mother via placenta. In infants born prematurely (before the 32^{nd} week of pregnancy) these immunoglobulines are nearly totally absent.

The only treatment of infections in the newborn infants that exists today is the use of antibiotics against bacterial infections and antimycotics against fungal infections. Such treatments have many drawbacks. Antibiotics and antimycotics are very often toxic, e.g. detrimental for the sense of hearing, for the neurologic system, for the kidneys and for the bone marrow, and the newborn infant is more susceptible to these toxic effects than older children and adults. Antibiotics may even cause alternative infections, especially fungal infections, which are more severe than the original infection. The most alarming drawback regarding antibiotics and antimycotics is, however, that several bacteria and fungi very rapidly are becoming resistant against these antimicrobial drugs. Thus, repetitive use of any antimicrobial agents is undesirable.

The natural response by humans to any infection is the production of antibodies of the IgG-class directed to systemic infections and secretory IgA directed to infections of the mucus membrane, including the oral cavity, the digestive tract and genitourinary tract. As the immunsystem is not mature to produce antibodies in the newborn, the ability to use an extraneous source of immunglobulines is a desirable objective in the case of treatment and prophylaxis in newborn infants.

Conventional extraneous sources of bulk polyclonal antibody are derived from serum of mammals after having immunised the mammal with a specific microbe. These polyclonal antibodies are antigenic in themselves and are therefore unsuitable for long term application in humans. The raise of anti-IgG antibodies will cause loss of activity of the IgG. Orally administered IgY does not raise any anti-IgY antibodies. Thus IgY will not loose activity even after having been used for a long time. The production of IgG antibodies requires a large number of animals, which have to be bled repeatedly. Thus there are also ethical and animal protection objections to the use of IgG.

In addition to prematurely born infants, new born infants and infants having an immature immune system, there are also other patients in need of new and improved treatments. These are for example patients suffering from temporary immunodeficiency or immunodeficiency diseases such as AIDS.

Yoshinori Mine et al (*Chicken egg yolk antibodies as therapeutics in enteric infectious disease: a review,* J Med Food, Vol. 5, no. 3, 2002, pages 159-167) describes the use of chicken egg yolk antibodies in treating enteric infections, such as those caused by *Escherichia coli.* Diarrhea has for example been treated in children.

Marquardt Ronald R. et al (*Passive protective effect of egg-yolk antibodies against enterotoxigenic Escherichia coli K88*+ *infection in neonatal and early-weaned piglets,* FEMS Immunol Med Microbiol, vol. 23, 1999, pages 283-288) describes the use of IgY from chicken eggs in treating *Escherichia coli* in neonatal and early-weaned piglets.

Neither of these documents mentions treatment of neonatal infants or immunodeficient patients, or treatment of infections caused by *Enterobacter cloacae.*

### Summary of the invention

In view of the drawbacks associated with prior art methods for treatment and prophylaxis of enteric infections in newborn infants, prematurely born infants, infants having an immature immune system, patients suffering from temporary immunodeficiency and immunodeficiency diseases such AIDS, there is a need for a new and improved method for treatment and prophylaxis of such infections.

The present invention relates to a novel method for treatment and prophylaxis of such enteric infections, which is both safe and effective. This is achieved by using IgY directed against enteric bacteria, and in particular against Enterobacter cloacae, and Candida albicans, which has been obtained from the egg yolk of birds, that have been hyperimmunised with an antigen (=microbe) in order to stimulate the production of immunoglobulines (IgY) against this microbe.

The conventional way of treating said patients with enteric infections has previously been by using antibiotics. This way has several disadvantages as mentioned above. The applicants' intensive studies of problems related to the gastrointestinal tract have led them to the surprising discovery that, due to the unique properties of newborn infants and prematurely born infants both regarding their gastrointestinal tract and immune system, a very efficient treatment of enteric infections in newborn infants, prematurely born infants and infants having an immature immune system can be achieved by using avian IgY directed against enteric bacteria. No one has previously considered this possibility.

The present invention also relates to a pharmaceutical product from eggs of birds containing immunoglobuline or a fraction thereof, which can be combined with other preparations or pharmaceuticals for simultaneous, separate or sequential use in the prophylaxis or therapy of gastrointestinal infections in newborn infants.

The present invention further relates to a pharmaceutical IgY product including a nutritional agent such as human breast milk or a substitute therefore. The present invention also pertains to a prophylaxis or therapy method including administration of IgY together with such a nutritional agent.

The present invention further relates to a pharmaceutical IgY product including a buffering agent. The present invention also pertains to a prophylaxis or therapy method including administration of IgY together with such a buffering agent.

The present invention relates to prophylaxis or therapy of all kind of infections in newborn infants, prematurely born infants, and infants having an immature immune system, patients suffering from temporary immunodeficiency and immunodeficiency diseases such AIDS. The infection can be any infection caused by an antigen such as bacterium, virus, fungus or parasite, that is infections in general, preferably bacterial infections, more preferably enteric infections and most preferably infections caused by Enterobacter cloacae and Candida albicans.

### Detailed description if the invention

The human infant in the first six months of life is particularly vulnerable to diarrhoea. The vulnerability to diarrhoeal processes is associated with the state of development of the gastrointestinal tract, not only of its digestive and absorptive processes but also of its unique and elaborate local defence system, consisting of both immune and non-immune elements. Protection from enteric infection, and from sensitisation to food antigen is a function of the integration of gastric acid and biliary secretion, intestinal motor activity and local immune mechanisms. Development of these functions is controlled by a species-specific program, in which the potential for accelerated development is very limited.

A newborn infant differs in many essential ways from older children and adults. As mentioned above, the immune system, especially regarding the B-cell-derived immunoglobulines, is very immature in the newborn infant's, and they have to rely on immunoglobulines that have been transported to them from their mother via placenta. Immunoglobulines are nearly totally absent in infants born prematurely before the 32^{nd} week of pregnancy.

The present invention is based on the discovery that anti-Enterobacter IgY has an unexpectedly good capacity to cure prematurely born newborn infants who have a very poor capacity to fight infections due to their immature immune system. The amount of gastric acid, which is an important factor in the protection against bacteria in the intestinal canal, is low in newborn infants. The unexpectedly good effect of the present invention is probably due to the relatively high pH in the stomach of the newborns, i.e. approximately 2-4. The normal pH of the stomach, i.e. approximately 1, would otherwise probably have inactivated the IgY.

IgY is the chicken antibody that corresponds to mammalian IgG. IgY consists of two light chains and two heavy chains and has a molecular weight of approximately 180 000 Da. IgY is actively transported from the hen to the egg and the egg yolk, which thus contains high concentrations of IgY.

One egg yolk contains around 100 - 200 mg of IgY antibodies. Most humans regularly consume ½ - 1½ egg per day and have achieved tolerance against proteins from eggs (including the immunoglobuline (IgY)). These patients will not get any allergic reaction when treated with IgY. Thus, there is no risk for an allergic response when treating these patients with IgY. However, patients with known egg allergy should not be treated with IgY. A dose in the order of 2 mg IgY would probably suffice to achieve the desired prophylactic or curing effects.

Hens, which have been immunised with microbes, respond by producing specific, polyclonal antibodies against the microbe. The antibodies can be purified from the egg yolk. Several in vitro studies show that bacterial, viral and fungal infections can be prevented with IgY. Many studies have also shown that peroral administration of specific IgY is used successfully to treat bacterial and viral infections in animals.

Compared to mammalian polyclonal antibodies IgY reacts with different epitopes on the antigen than the mammalian antibodies do. This gives access to a different antibody repertoire than the mammalian antibody. The mode of action of the specific antibody is related to the number of organisms present at a given moment. It will be appreciated that there is a direct molecular correlation between antibody entities attaching to each microbe and the numbers of microbes present. The dose level will also be related to the total surface area of affected tissue and biological parameters which affect "wash out" ratios.

When the immunoglobuline is to be administered by the oral route, it will preferably contain a buffering agent to prevent deactivation at low pH-values which can optionally be administered in the form of a nutritional complement.

IgY antibodies also have biochemical properties that make them advantageous over IgG for peroral immunotherapy: They neither activate the human complement system nor react with rheumatoid factors, human anti-mouse IgG antibodies (HAMA) or human Fc-receptors. Those are all well-known cell activators and mediators of inflammation.

As IgY is a normal dietary component there is no risk of toxic reactions in the patient, except for those who have a known allergy to eggs.

Egg immunoglobuline is classified as avian IgY which is similar to mammalian secretory IgA, and therefore a natural part of the mucus epithelial environment.

One of the objects according to the invention is to provide a pharmaceutical composition from eggs of birds comprising immunoglobuline or a fraction thereof for use in the prophylaxis or therapy of infections in the newborn infant.

As indicated above, the infections in newborn infants can be attributed to a multiplicity of factors, including long-term exposure to antibiotics, which disrupt the normal balance of the intestinal micro-flora. The preparation will be designed to reinstitute the normal balance of the microflora of the newborn infants. The formulations according to the present invention can be used as an alternative or supplement to such treatments.

According to a basic embodiment of the present invention, by mixing the IgY according to the present invention with any pharmaceutically acceptable carrier or diluent, a pharmaceutical composition or medicament is obtained. The medicament containing IgY can be formulated as a freeze dried or lyophilised powder, a solution, a lozenge, a tablet or as a capsule. This pharmaceutical medicament is preferably administered in a form suitable for oral administration. together with any other pharmaceutically acceptable carrier or diluent.

In another embodiment the pharmaceutical medicament according to the present invention is formulated as a controlled or sustained release formulation.

According to another embodiment of the present invention, the IgY can be administered without any conventional diluent or recipient in a nutritional agent such as human breast milk or a substitute therefore.

During their experiments, the present inventors have found that the combination of human breast milk and IgY, administered as an emulsion, protects the IgY conserves its activity. The present inventors believe that this is due to the emulsion that is formed from IgY and the milk. This can also be an effect of the buffering ability of the milk, which enhances the lifetime of the IgY.

One embodiment of the present invention relates to a method of prophylaxis or treatment of newborn infants having a pH in their stomachs above 1,5, particularly between 1,5 and 4. This method comprises administrating IgY, that originates from an egg of a bird hyperimmunised with a microbe, to the infant. The method particularly relates to prophylaxis or treatment of prematurely born infants, and more particularly to infants having a weight below 2500g.

In the present application different terms have been used which will be defined below.

By the terms "immunoglobuline" or "fragment of an immunoglobuline" is meant an antibody, or antibody fragment, or antibody precursor capable of binding to a specific microbe or fragment thereof so as to render it non-pathogenic.

The pharmaceutical product according to the present invention can also be used in conjunction with, or include, an antimicrobial agent of the kind used in conventional therapy of infections of the newborn infants.

The products and methods of IgY for prophylaxis or treatment according to the present invention have also been working on patients suffering from temporary immunodeficiency, e.g. immunosuppressed patients with leukaemia have been treated successfully with anti-candida IgY. Said administration relates to oral application in order to prevent or treat oral and pharyngeal candidiasis infections caused by Candida albicans. Said type of administration for the purpose of preventing or treating oral and pharyngeal infections is disclosed in an another application filed previously by the same inventors is not part of the subject-matter of the present invention. However, early studies of administering anti-candida IgY to immunosuppressed patients in order to remove candida from the gastrointestinal canal in order to avoid enteric infections and infections in other parts of the body, such as the vagina, seems promising. In order to treat enteric infections, as in the case of anti-Enterobacter IgY, the anti-candida IgY needs to be buffered or combined with a nutritional agent according to the present invention, if not administered to a newborn or a prematurely born infant. Thus, the present invention also relates to prophylaxis or treatment of patients with temporary immunodeficiency and immunodeficiency diseases such as AIDS.

### Examples

### Preparation of Enterobacter cloacae

Enterobacter cloacae isolated from faeces of infected newborns was used in an in vitro experiment to demonstrate the prophylactic potential of egg immunoglobuline isolated from domestic hens hyper-immunised with Enterobacter cloacae antigen.

The bacteria were grown in 500-ml flasks containing 100 ml of 2% glucose, 0.15% yeast nitrogen base, 0.5% ammonium sulphate supplemented, where necessary, with amino acids. The flasks were shaken at 200 r.p.m. in a rotary incubator at 37°C for 24 hours.

### Preparation of anti-Enterobacter cloacae IgY immunoglobuline.

A suspension of formalin-killed Enterobacter cloacae was washed in saline and freeze-dried in ampoules; 2.0 x 108 bacterial cells per ampoule. Twice a week, inoculations were carried out intramuscularly in domestic hens, using 1.0 ml purified water to resuspend each ampoule. Yolks of eggs collected from hyper-immunised hens were assayed to determine peak antibody titre using an ELISA (enzyme linked immunosorbent assay) specific for Enterobacter cloacae IgY.

After 3 to 4 weeks, when peak titre had been achieved, egg yolks were harvested by separation from the egg white and removing the contents of the yolk sac using a hypodermic syringe. The immunoglobuline fraction was purified using an industrial standard of supercritical CO₂-equipment to dissolve the lipid. Leaving the proteinaceous polyclonal immunoglobuline in a purified state. The immunoglobuline fraction was diluted using distilled water to a concentration of 10 mg/ml and lyophilised in trays. This solution was used to evaluate the prophylactic potential of the anti-Enterobacter IgY indicated below in the example *Treatment of newborn infants with anti-Enterobacter IgY.*

### Cell adhesion assay

Adhesion to mucus epithelium cells is considered to be the primary stage in infection. In this example epithelial cells and pseudomonas aeruginosa (as representative for bacteria) were used to evaluate adhesion.

Fresh cells cultured for 24 hour were washed by centrifugation in PBS, re-suspended in PBS and mixed with medium containing either IgY of eggs from hens immunised with pseudomonas or IgY from non-immunised hens at a ratio of 100:1 or no IgY at all and incubated at 37°C for 2 hours. After incubation, the culture was filtered through a 45gm filter to remove any unadhered cells, washed in PBS and re-suspended.

The adherence was evaluated by microscopic examination at 400 magnifications using a stage micrometer grid to facilitate accurate counting. Adherence was expressed as a percentage of cells with visibly adhering pseudomonas aeruginosa bacteria.

The results showed that the adhesion of *P. aeruginosa* to epithelial cells was reduced by more than 50% in the case of bacteria treated with immunoglobuline from immunised hens, when compared with both untreated bacteria and bacteria treated with extract of normal egg.

The above in vitro experiments demonstrate that purified immunoglobuline fractions extracted from the yolk sac of eggs laid by hens previously hyperimmunised with P. aeruginosa antigen inhibit epithelial cell adhesion. These experiments enables one to conclude that specific egg immunoglobuline can be used in the prophylaxis or therapy of infections in the newborn infants.

### Treatment of newborn infants with anti-Enterobacter IgY

Infections caused by Enterobacter chloacae are extremely dangerous for prematurely born infants. According to the literature 20 - 50% of premature babies of this weight, who gets an infection with Enterobacter chloacae, will die or get severe sequels.

In the experiment anti-Enterobacter IgY was used to treat prematurely born infants of less than 2500g, who have been infected by Enterobacter chloacea. Ten infants were daily given IgY with a high specific titre against Enterobacter in human breast milk. The treatment started as soon as a culture from stools or blood had been positive for Enterobacter chloacae. In nine of these patients Enterobacter chloacae was eradicated from their stools. Only one patient, who already had a bacteraemia with Enterobacter before the treatment started, continued to have stool and blood cultures positive for Enterobacter for nearly a month. None of the patients died and none of them got any sequel. None of them developed any allergy to eggs.

The invention is not limited to the embodiments described above which may be modified and/or varied without departing from the scope of the invention.

## Claims

1. A use of IgY that originates from an egg of a bird hyperimmunised with Enterobacter cloacae for the production of a pharmaceutical for treatment and/or prevention of enteric infections in newborn infants, prematurely born infants, infants having an immature immune system, patients suffering from temporary immunodeficiency and immunodeficiency diseases such as AIDS.

2. The use according to claim 1, wherein the IgY is formulated as a freeze dried or lyophilised powder, a solution, an emulsion, a lozenge, a tablet or as a capsule together with any other pharmaceutically acceptable carrier or diluent.

3. The use according to claims 1 or 2, wherein the pharmaceutical further comprises a buffering agent.

4. The use according to any of claims 1-3, further comprising a nutritional agent.

5. The use according to claim 3 or 4, wherein the nutritional agent or buffering agent is human breast milk or a substitute therefore.

6. The use according to any of the preceding claims, wherein the enteric infection is an intestinal infection.

7. The use according to any of the preceding claims, wherein said newborn infants or prematurely born infants have a weight below 2500g.

8. The use according to any of the preceding claims, wherein said newborn infants have a pH value between 1,5 and 4 in their stomach.

9. A pharmaceutical composition comprising IgY that originates from an egg of a bird hyperimmunised with Enterobacter cloacae.

10. The pharmaceutical composition according to claim 9, wherein the pharmaceutical composition is formulated as a freeze dried or lyophilised powder, a solution, an emulsion, a lozenge, a tablet or as a capsule together with any other pharmaceutically acceptable carrier or diluent.

11. The pharmaceutical composition according to claim 9 or 10, wherein the pharmaceutical further comprises a buffering agent.

12. The pharmaceutical composition according to any of claims 9-11, further comprising a nutritional agent.

13. The pharmaceutical composition according to claim 11 or 12, wherein the nutritional agent or buffering agent is human breast milk or a substitute therefore.

## Patentansprüche

1. Verwendung von IgY, das von einem Ei eines Vogels stammt, der mit *Enterobacter cloacae* hyperimmunisiert wurde, zur Herstellung eines Pharmazeutikums für die Behandlung und/oder Prävention von enterischen Infektionen bei neugeborenen Kindern, vorzeitig geborenen Kindern, Kindern mit einem unausgereiften Immunsystem, Patienten, die unter temporärer Immunschwäche und Immunschwächeerkrankungen wie AIDS leiden.

2. Verwendung nach Anspruch 1, wobei das IgY als gefriergetrocknetes oder lyophilisiertes Pulver, Lösung, Emulsion, Pastille, Tablette oder Kapsel zusammen mit irgendeinem anderen pharmazeutisch annehmbaren Träger oder Verdünnungsmittel formuliert ist.

3. Verwendung nach Anspruch 1 oder 2, wobei das Pharmazeutikum ferner ein Pufferungsmittel umfasst.

4. Verwendung nach irgendeinem der Ansprüche 1 bis 3, welche ferner ein Nährmittel umfasst.

5. Verwendung nach Anspruch 3 oder 4, wobei das Nährmittel oder das Pufferungsmittel humane Muttermilch oder ein Ersatz dafür ist.

6. Verwendung nach irgendeinem der vorhergehenden Ansprüche, wobei die enterische Infektion eine Darminfektion ist.

7. Verwendung nach irgendeinem der vorhergehenden Ansprüche, wobei die neugeborenen Kinder oder vorzeitig geborenen Kinder ein Gewicht unter 2500 g aufweisen.

8. Verwendung nach irgendeinem der vorhergehenden Ansprüche, wobei die neugeborenen Kinder einen pH-Wert zwischen 1,5 und 4 in ihrem Magen aufweisen.

9. Pharmazeutische Zusammensetzung, umfassend IgY, das von einem Ei eines Vogels stammt, der mit *Enterobacter cloacae* hyperimmunisiert wurde.

10. Pharmazeutische Zusammensetzung nach Anspruch 9, wobei die pharmazeutische Zusammensetzung als gefriergetrocknetes oder lyophilisiertes Pulver, Lösung, Emulsion, Pastille, Tablette oder Kapsel zusammen mit irgendeinem anderen pharmazeutisch annehmbaren Träger oder Verdünnungsmittel formuliert ist.

11. Pharmazeutische Zusammensetzung nach Anspruch 9 oder 10, wobei das Pharmazeutikum ferner ein Pufferungsmittel umfasst.

12. Pharmazeutische Zusammensetzung nach irgendeinem der Ansprüche 9 bis 11, welche ferner ein Nährmittel umfasst.

13. Pharmazeutische Zusammensetzung nach Anspruch 11 oder 12, wobei das Nährmittel oder das Pufferungsmittel humane Muttermilch oder ein Ersatz dafür ist.

## Revendications

1. Utilisation d'un IgY qui provient d'un oeuf d'un oiseau hyperimmunisé avec Enterobacter cloacae pour la production d'un produit pharmaceutique permettant le traitement et/ou la prévention d'infections entérales chez les nouveaux-nés, les prématurés, les nourrissons ayant un système immunitaire immature, les patients souffrant d'immunodéficience temporaire et de maladies immunodéficientes telles que le SIDA.

2. Utilisation selon la revendication 1, dans laquelle le IgY est formulé sous la forme d'une poudre lyophilisée, d'une solution, d'une émulsion, d'une pastille, d'un comprimé ou d'une capsule avec tout autre porteur ou diluant pharmaceutiquement acceptable.

3. Utilisation selon les revendications 1 ou 2, dans laquelle le produit pharmaceutique comprend en outre un agent de tamponnement.

4. Utilisation selon l'une des revendications 1 à 3, comprenant en outre un agent nutritif.

5. Utilisation selon la revendication 3 ou 4, dans laquelle l'agent nutritif ou l'agent de tamponnement est du lait maternel humain ou un substitut de celui-ci.

6. Utilisation selon l'une des revendications précédentes, dans laquelle l'infection entérale est une infection intestinale.

7. Utilisation selon l'une des revendications précédentes, dans laquelle lesdits nouveaux-nés ou prématurés ont un poids inférieur à 2500 g.

8. Utilisation selon l'une des revendications précédentes, dans laquelle lesdits nouveaux-nés présentent une valeur de pH entre 1,5 et 4 dans leur estomac.

9. Composition pharmaceutique comprenant un IgY qui provient d'un oeuf d'un oiseau hyperimmunisé avec Enterobacter cloacae.

10. Composition pharmaceutique selon la revendication 9, dans laquelle la composition pharmaceutique est formulée sous la forme d'une poudre lyophilisée, d'une solution, d'une émulsion, d'une pastille, d'un comprimé ou d'une capsule avec tout autre porteur ou diluant pharmaceutiquement acceptable.

11. Composition pharmaceutique selon la revendication 9 ou 10, dans laquelle le produit pharmaceutique comprend en outre un agent de tamponnement.

12. Composition pharmaceutique selon l'une des revendications 9 à 11, comprenant en outre un agent nutritif.

13. Composition pharmaceutique selon la revendication 11 ou 12, dans laquelle l'agent nutritif ou l'agent de tamponnement est du lait maternel humain ou un substitut de celui-ci.
